# EUROPEAN PATENT SPECIFICATION

(11) **EP 0 757 548 B1**
(45) Date of publication and mention of the grant of the patent: **14.01.1998**
(21) Application number: 95918589.3
(22) Date of filing: 21.04.1995
(51) Int. Cl.: A61F 6/00

(54) **INTRAUTERINE DEVICE TRAY HAVING ONE ATTACHMENT SITE**
TRÄGER MIT EINER VERBINDUNGSSTELLE FÜR EINE INTRAUTERINE VORRICHTUNG
SUPPORT D'INTRODUCTION D'UN DISPOSITIF INTRA-UTERIN, POURVU D'UN SITE DE FIXATION

(30) Priority: 25.04.1994 EP 94201133
(43) Date of publication of application: 12.02.1997
(73) Proprietor: Akzo Nobel N.V., 6824 BM Arnhem (NL)
(72) Inventor: GILLISSEN, Johannis, NL-5345 BW Oss (NL)
(74) Representative: Kraak, Hajo
(86) International application number: EP9501525
(87) International publication number: WO9528901

(56) References cited:
- EP-A- 0 049 660
- EP-A- 0 191 957
- EP-A- 0 584 628
- US-A- 3 965 891
- US-A- 4 111 302
- US-A- 4 549 652

## Description

The invention relates to an IUD-tray comprising an attachment device having one attachment site which fits to an adjustable stop on a sheath, which sheath may be attached to an IUD (intrauterine device), and to a combination of the IUD and said IUD-tray.

IUD's (intrauterine devices) are inserted into the uterus through the cervix. To obtain the required effect it is necessary to insert the IUD in such a manner that the top of the IUD just touches the uterine wall, but on insertion damage of the uterine wall must be prevented. Most IUD's therefore, are mounted on a insertion device (injector), usually a tabular instrument or a sheath, provided with means for adjusting the required insertion depth. Such means is usually a stop which can slide over the insertion device (for instance a sheath) to adjust the required insertion depth of the IUD. Such stop is usually executed as a slidable ring. The IUD attached to the insertion device is introduced into the uterine cavity until the stop touches the fundus of the uterus, after which the insertion device with the stop is removed.

In order to keep the IUD sterile and undamaged, it is important to use a method of adjustment of the insertion depth of the IUD which circumvents considerable handling of the IUD prior to introduction into the uterine cavity. A method which does not require pushing the IUD, which might cause damage to the uterine wall, is preferred. Such method is, for instance, used for the IUD disclosed in USP 3,952,734, which is commercially available under the trademark Multiload®. According to this method the IUD is inserted into the uterine cavity by means of a tube-shaped sheath, having an adjustable stop, which sheath is attached to the IUD (in such a manner that sheath and IUD loosely fit). The stop can be slid on the sheath to obtain the required insertion depth for the IUD.

In the most simple embodiment according to the art, the IUD attached to the sheath with the adjustable stop is mounted on a cardboard and the adjustable stop is slid to the required distance from the top of the IUD. The adjustment is performed by estimating the distance or by using a separate ruler, which method has the disadvantage that the IUD can be contaminated and desterilised. There is, however, a need to adjust the required distance more accurately. Therefore, IUD-trays having devices to determine the required distance are proposed.
A typical IUD-tray having such device, is the IUD-tray in which Multiload® is packed, which is commercially available from Multilan AG, Switzerland. This IUD-tray has an attachment device which is part of the IUD-tray, and is used to adjust the distance of the top of the IUD to the adjustable stop to a pre-determined distance corresponding to the required insertion depth of the IUD. The adjustable stop is clamped into one of the attachment sites of the attachment device and the sheath is pulled or pushed, after which the adjustable stop slides to the pre-determined distance on the sheath, which sheath is attached to the IUD.
In EP 584,628 an IUD-tray has been disclosed which can contain an IUD mounted on a shaft, which has an adjustable stop. The IUD-tray further has a ruler as measuring device for the adjustment of the stop. The IUD-tray according to this reference does however not have an attachment device having an attachment site which fits to the stop. Although this device allows accurate adjustment of the stop, the stop must be adjusted to the desired distance from the top of the IUD, which method still has the disadvantage that the IUD can be contaminated and desterilised.
The attachment device of the IUD-tray of the Multiload® IUD has three or four rectangular attachment sites into which the adjustable stop can be placed. When the stop is placed into one of the sites, the sheath can be slid upwards and downwards, keeping the stop fixed at the attachment site, to adjust the insertion depth of the IUD. The sheath is pulled or pushed until the IUD fits into a compartment for containing the IUD. By choosing one of the three or four attachment sites, an insertion depth of respectively 5, 6, and 7 cm, or 6, 7, 8, and 9 cm can be obtained.

This device, although an improvement in the art, has some disadvantages. To pull (or push) the sheath through the adjustable stop, the sheath carrying the IUD must be lifted out of the IUD-tray in order to attach the adjustable stop into the required attachment site.
Typically the IUD then extends over the upper part of the IUD-tray and will be pulled back until it drops into the compartment in which the IUD fits. The IUD thus moves over the upper part, the rim, or the surface of the IUD-tray. The disadvantage of this method is that the IUD can be damaged and that the copper wire on the stem of the IUD can be uncovered. Further, the IUD, by touching non-sterile objects in its surrounding, may be desterilised.
Usually, as for the Multiload® IUD, a pulling wire is attached to the stem of the IUD, which wire is pulled through the interior of the hollow sheath and extends from the end of the sheath. The pulling wire should firmly be held by the physician who inserts the IUD to prevent the sheath being pulled prematurely from the IUD. There is considerable chance that the IUD drops from the sheath when the pulling wires are not firmly held, particularly when the arms of the IUD touch the rim of the IUD-tray. The necessity to firmly hold the pulling wires during adjustment of the insertion depth is an additional burden for the physician.
Another disadvantage of the IUD-tray of the art is the restricted number of insertion depths that can be adjusted, since the insertion depth of the sheath can only be measured in discrete stages (i.e. for Multiload® only 5, 6, and 7 cm, or 6, 7, 8 and 9 cm), whereas there is a need for a more accurate adjustment, possibly over a wider range. This IUD-tray further necessitates the production of two different packagings to obtain IUD's for shorter and for longer insertion depths.

It is an object of the present invention to provide an IUD-tray which solves the above-mentioned disadvantages. In the broadest sense the present invention concerns an IUD-tray, suitable for containing an IUD attached to a sheath with an adjustable stop, comprising an attachment device, characterized in that the attachment device has a single attachment site which fits to the adjustable stop on the sheath and in that the IUD-tray has an IUD compartment which has a form to permit the IUD to shift in order to adjust the top of the IUD at the required distance from the adjustable stop, to facilitate the adjustment of the adjustable stop in a sterile manner. Preferably the IUD-tray is further provided with a ruler, more particularly which is positioned in the portion of the IUD-tray for containing the portion of the sheath comprising the adjustable stop.

For reasons of sterility and convenience it is preferred that the device to slide the ring forms an integral part with the IUD-tray in which the IUD is packed. In a preferred embodiment the invention therefore concerns an IUD-tray having a ruler and an attachment device which fits to an adjustable stop on a sheath which can be attached to an IUD, wherein this attachment device has one attachment site.

The term sheath means any injector with which the IUD is inserted into the uterus. The sheath can be tubular or tube-shaped, but preferably the sheath is a hollow tube in which the stem of the IUD is inserted. The sheath may have any form, such as triangular, hexagonal, or octagonal, but preferably the sheath is oval or round. Preferably pulling wires attached to the stem of the IUD are led through the interior of the sheath.
The adjustable stop should be a stop which can be slid over the sheath. The stop can have any form, but preferably the form is an oval or ring form. The oval or ring formed stop may be flattened at one or more sides to allow the stop to remain more stable in the IUD-tray.

The IUD may be any IUD which can be attached to a sheath. A preferred IUD is an IUD having two resilient cantilevered arms, like the IUD described in USP 3,952,734.

The attachment site has a form which fits to the adjustable stop. For instance, when the stop contains a stud, the attachment site should contain a corresponding cavity which fits to the stud of the stop. Alternatively, the attachment site may contain a stud or other means which fit into a cavity of the stop. Preferably however, the attachment site is a cavity, which fits to the stop. With more preference the attachment site has a shape which not only fits to the adjustable stop, but moreover clamps the stop. This prevents the IUD from falling out of the IUD-tray.

The IUD-tray is preferably provided with a ruler to supply means for reading the required distance of the top of the IUD to the adjustable stop. The measure can be given by indication lines in any feasible unit, for instance in inches or centimetres. The ruler can be carried out on the IUD-tray by any method such as by painting or printing the ruler, or attaching a sticker with said ruler, or likewise. Preferably the ruler is carried out as a relief in the IUD-tray. The ruler can be placed at any portion of the IUD-tray for instance at the part which is suitable for containing the end of the sheath (the side of the sheath opposite to the side to which the IUD is attached), whereby this end is used to measure the required distance from the top of the IUD to the adjustable stop. With more preference however, the ruler is introduced in the portion of the IUD-tray which is suitable for containing the IUD, whereby the top of the IUD is used to measure the required distance directly. It is of advantage to carry out the ruler with indication lines which are readable on both sides of the sheath, when the sheath is in the IUD-tray.

When the sheath is moved upwards or downwards in the longitudinal direction of the IUD-tray, the end of the sheath may extend from the IUD-tray. In order to easily move the sheath it is preferable to bevel the IUD-tray at the lower side of the portion of the IUD-tray which is suitable for containing the end of the sheath. When the IUD-tray is performed with a bevelled side, the end of the sheath can then smoothly be slipped over this side.

In another embodiment the attachment device can be carried out as a slidable attachment device. Using this embodiment, the sheath with the attached IUD is fixed in the IUD-tray, and the attachment device, attached through its attachment site to the adjustable stop, is slid in the IUD-tray to move the stop on the sheath to the required distance from the top of the IUD.

It is also preferred to provided the IUD-tray with a relief on the inside of the portion of the IUD-tray which is suitable for containing the end of the sheath.
This relief gives grip to the fingers during adjustment of the stop. The relief may for instance be in the form of a text such as a brand name or type indication.

It is useful to make the IUD-tray of resilient material to improve clamping of the attachment device to the adjustable stop and to facilitate removal of the stop from the attachment site when adjusted. Preferably the material is furthermore transparent to facilitate inspection of the IUD in a closed IUD-tray. Suitable materials for the manufacture of the IUD-tray are known in the art, for instance cardboard or plastics such as polypropylene, polyethylene, polycarbonate, polystyrene, and PETG (polyethylene terephthalate glycol comonomer).

The dimensions of the IUD-tray are preferably chosen to allow enough space for the fingers to fetch the sheath.
This is usually attained when the longitudinal sides of the IUD-tray have a distance of about 0.75-4 cm to the sheath.

Most preferred is an IUD-tray in which the attachment device is positioned at about the middle of the IUD-tray, and the ruler is supplied in such a way that the indication, lines can be read at both sides of the sheath when the sheath is in the IUD-tray.

A particular useful IUD-tray is the IUD-tray wherein (a) the attachment device is positioned at about the middle of the IUD-tray; (b) the portion for containing the IUD has a form in which the IUD can freely move; (c) the ruler can be read at both sides of the sheath when the sheath is in the IUD-tray; and (d) the breadth of the IUD-tray is about 1.5-8 cm.

The IUD-tray can be covered by any kind of lid, preferably by a peelable sheet. This type of covering requires less packing material. The cover can be of any suitable material such as plastic, cardboard, paper, or plastified paper.

The IUD-tray containing the IUD and sheath, possibly closed by a lid, can be sterilised in the usual manner, for instance by treatment with ethylene oxide.

It is another object of the invention to provide a combination comprising an IUD and an IUD-tray wherein the IUD can be any IUD known in the art and the IUD-tray is an IUD-tray according to this invention. Preferably the IUD of the combination has two resilient cantilevered arms curving back towards the stem of the IUD. The combination further comprises the sheath attached to the IUD.

The invention is further illustrated by the figures, without being restricted to the particular embodiment of these figures.
- Fig. 1: shows an IUD attached to a sheath with adjustable stop.
- Figs. 2 and 3: show the IUD with sheath of Fig. 1 at different insertion depths.
- Fig. 4: shows a top view of a part of an IUD-tray known in the art with an attachment device with four attachment sites.
- Fig. 5: shows a top view of the combination of an IUD-tray according to this invention, a sheath with an adjustable stop, and an IUD.
- Fig. 6: shows a side view of the combination of an IUD-tray according to this invention, a sheath with an adjustable stop, and an IUD.
- Figs. 7 and 8: show a side view of the combination of an IUD-tray according to this invention, a sheath with an adjustable stop, and an IUD at different insertion depths.

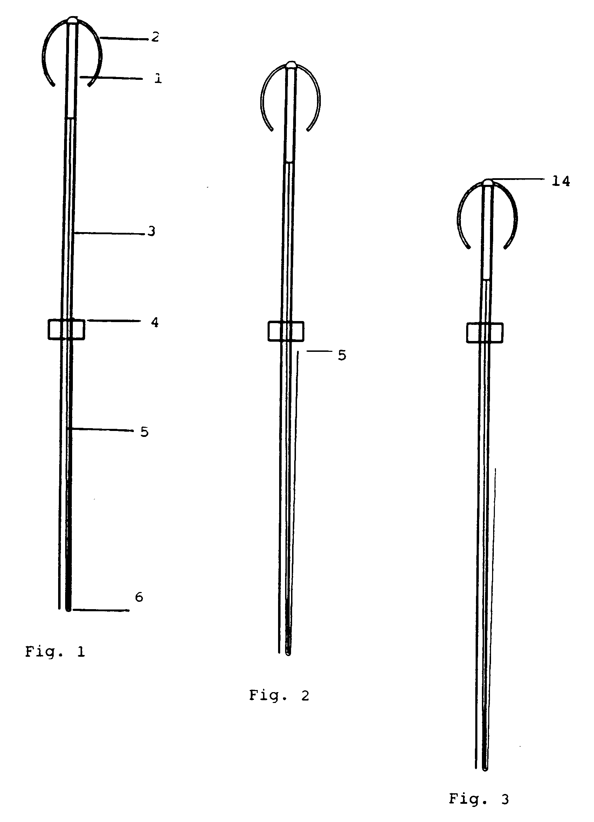

In Fig. 1 an IUD (1) is shown with arms (2) attached to a tube-shaped sheath (3) with an adjustable stop (4), and a pulling wire (5) which is attached to the IUD and is led through the hollow tube-shaped sheath and which extends from the end of the sheath (6).

The same IUD with sheath is shown in Figs. 2 and 3, however the adjustable stop is shifted to positions which correspond to required insertion depth (Fig. 2 showing a greater insertion depth than Fig. 3).

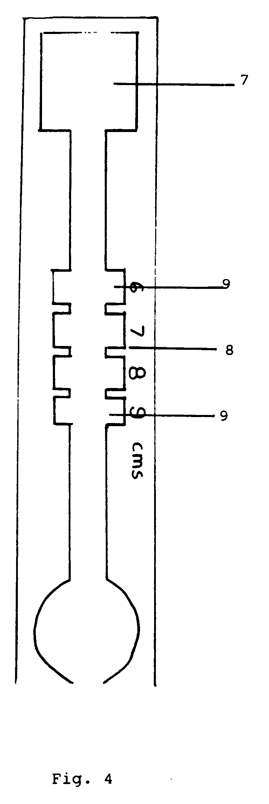

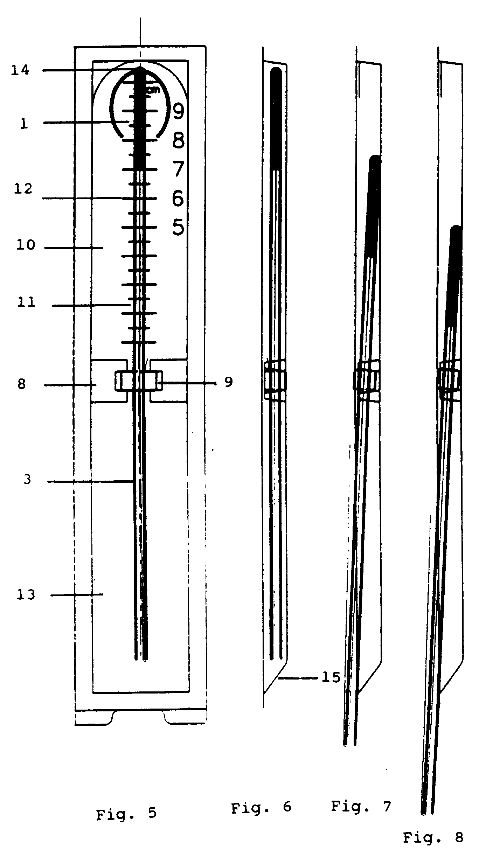

A part of an IUD-tray of the art as used for the IUD of USP 3,952,734 is seen in Fig. 4. This IUD-tray comprises a compartment (7) suitable for containing the IUD (1) and the attachment device (8) comprising four attachment sites (9). In the method of the art the adjustable stop (4) on the sheath (3) is clamped into one of the four attachment sites, after which the sheath is pulled until the IUD drops into compartment (7). The pulling wire (5) must firmly be held during this procedure to prevent the sheath being detached from the IUD.
An IUD-tray according to this invention which contains the IUD (1) attached to the sheath (3) is shown in Fig. 5, the IUD-tray having an IUD compartment (10) which has a form to permit the IUD to shift downwards, a ruler (11) with indication lines (12), an attachment device (8) having one attachment site (9), and a portion of the IUD-tray which is suitable for containing the end of the sheath (13). The stop of the sheath can be clamped into the attachment site, after which the sheath can be pulled over a distance until the top (14) of the IUD reaches a pre-determined value on the ruler.

Fig. 6 shows a side view of the IUD-tray of Fig. 5 wherein the IUD and sheath are clamped through the adjustable stop into the attachment site. The IUD-tray has a bevelled side (15) at the lower side of the portion of the IUD-tray which is suitable for containing the end of the sheath.

Figs. 7 and 8 show two different positions of the IUD when it has been pulled backwards in the IUD-tray. Since the arms of the IUD cannot touch the rim of the IUD-tray and thus cannot hook to that rim, it is not necessary to hold the pulling wires when pulling the sheath.

## Claims

1. An IUD-tray, suitable for containing an IUD (1) attached to a sheath (3) with an adjustable stop (4), comprising an attachment device (8), characterized in that the attachment device (8), has a single attachment site (9) which fits to the adjustable stop (4) on the sheath (3) and in that the IUD-tray has an IUD compartment (10) which has a form to permit the IUD (1) to shift in order to adjust the top of the IUD at the required distance from the adjustable stop (4).

2. The IUD-tray of claim 1, wherein the attachment site (9) is a cavity which fits to the adjustable stop (4).

3. The IUD-tray of claim 1 or 2, wherein the attachment site (9) has a shape which clamps to the adjustable stop (4).

4. The IUD-tray of any one of claims 1-3, wherein the IUD-tray further comprises a ruler (11).

5. The IUD-tray of claim 4, wherein the ruler (11) is carried out as a relief in the IUD-tray.

6. The IUD-tray of claim 4 or 5, wherein the ruler (11) is positioned in the portion of the IUD-tray which is suitable for containing the IUD (1), and preferably in the portion which is suitable for containing the portion of a sheath (3) which comprises the adjustable stop (4).

7. The IUD-tray of any one of claims 1-6, wherein the IUD-tray is bevelled at the portion of the IUD-tray which is suitable for containing the end (13) of the sheath.

8. The IUD-tray of any one of claims 1-7, wherein the IUD-tray has a relief on the inside of the portion of the IUD-tray which is suitable for containing the end (13) of the sheath.

9. The IUD-tray of any one of claims 1-8, wherein the IUD-tray is resilient, and preferably transparent material.

10. The IUD-tray of any one of claims 4-9, wherein (a) the attachment device (8) is positioned at about the middle of the IUD-tray; (b) the ruler (11) can be read at both sides of the sheath (3) when the sheath (3) is in the IUD-tray; and (c) the breadth of the IUD-tray is about 1.5-8 cm.

11. A combination comprising an IUD (1) and a tray, characterized in that the IUD (1) is any IUD known in the art and the tray is the IUD-tray as claimed in any one of claims 1-10.

12. The combination of claim 11, wherein the IUD (1) is an IUD having two resilient cantilevered arms (2) curving back towards the stem of the IUD.

13. The combination of claim 11 or 12, wherein the IUD (1) is attached to a sheath (3).

14. The combination of any one of claims 11-13, wherein the IUD-tray is covered, preferably by a peelable sheet.

## Patentansprüche

1. IUP-Träger zum Aufnehmen eines IUP (1), das mit einer verstellbaren Arretierung (4) an eine Hülle (3) angebracht ist, welche Arretierung ein Befestigungsmittel (8) umfasst, dadurch gekennzeichnet, dass das Befestigungsmittel (8) eine einzige Befestigungsstelle (9) aufweist, die zu die verstellbare Arretierung (4) auf der Hülle (3) passt, und dass der IUP-Träger über ein IUP-Fach (10) mit einer solchen Form verfügt, dass das IUP (1) sich verschieben kann, so dass das obere Ende des IUP im nötigen Abstand zu der verstellbaren Arretierung (4) eingestellt werden kann.

2. IUP-Träger nach Anspruch 1, worin die Befestigungsstelle (9) ein Hohlraum ist, der zu der verstellbaren Arretierung (4) passt.

3. IUP-Träger nach Anspruch 1 oder 2, worin die Befestigungsstelle (9) eine Form aufweist, die sich an die verstellbare Arretierung (4) klemmt.

4. IUP-Träger nach einem der Ansprüche 1 bis 3, worin der IUP-Träger ausserdem ein Lineal (11) umfasst.

5. IUP-Träger nach Anspruch 4, worin das Lineal (11) erhaben auf dem IUP-Träger angebracht ist.

6. IUP-Träger nach Anspruch 4 oder 5, worin das Lineal (11) an dem Teil des IUP-Trägers liegt, der sich zum Aufnehmen des IUP (1) eignet, und vorzugsweise an dem Teil, der sich zum Aufnehmen des Hüllenteils (3) eignet, der die verstellbare Arretierung (4) umfasst.

7. IUP-Träger nach einem der Ansprüche 1 bis 6, worin der IUP-Träger am Teil, der sich für das Aufnehmen des Hüllenendes (13) eignet, abgeschrägt ist.

8. IUP-Träger nach einem der Ansprüche 1 bis 7, worin der IUP-Träger an der Innenseite des IUP-Trägers eine Vertiefung aufweist, die sich zur Aufnahme des Hüllenendes (13) eignet.

9. IUP-Träger nach einem der Ansprüche 1 bis 8, worin der IUP-Träger aus elastischem und vorzugsweise durchsichtigem Material besteht.

10. IUP-Träger nach einem der Ansprüche 4 bis 9, worin (a) das Befestigungsmittel (8) etwa in der Mitte des IUP-Trägers liegt, (b) das Lineal (11) auf beiden Seiten der Hülle (3) abgelesen werden kann, wenn die Hülle (3) in dem IUP-Träger liegt; und (c) die Breite des IUP-Trägers etwa 1,5 - 8 cm beträgt.

11. Kombination umfassend ein IUP (1) und ein Träger, dadurch gekennzeichnet, dass das IUP (1) ein bekanntes IUP ist und der Träger die IUP-Träger ist, wie sie in einem der Ansprüche 1 bis 10 beansprucht ist.

12. Kombination nach Anspruch 11, worin das IUP (1) ein IUP mit zwei elastischen abstehenden Armen ist, die sich zurück in Richtung des IUP-Stammes biegen.

13. Kombination nach Anspruch 11 oder 12, worin das IUP (1) an eine Hülle (3) angebracht ist.

14. Kombination nach einem der Ansprüche 11 bis 13, worin der IUP-Träger vorzugsweise durch eine lösbare Schicht abgedeckt ist.

## Revendications

1. Un porte-stérilet susceptible de recevoir un stérilet (1) fixé à une gaine (3) par l'intermédiaire d'une butée réglable (4) comprenant un dispositif de fixation (8), caractérisé en ce que le dispositif de fixation (8) comporte un site de fixation unique (9) qui s'adapte à la butée réglable (4) sur la gaine (3) et en ce que le porte-stérilet comporte un logement de stérilet (10) qui a une forme permettant au stérilet (1) de coulisser de manière à permettre le réglage de l'extrémité du stérilet à la distance voulue par rapport à la butée réglable (4).

2. Le porte-stérilet selon la revendication 1 dans lequel le site de fixation (9) est une cavité qui s'ajuste à la butée réglable (4).

3. Le porte-stérilet selon la revendication 1 ou 2, dans lequel le site de fixation (9) présente une forme qui bloque la butée réglable (4).

4. Le porte-stérilet selon l'une quelconque des revendications 1 à 3, dans lequel le porte-stérilet comprend en outre une réglette (11).

5. Le porte-stérilet selon la revendication 4, dans lequel la réglette (11) se présente sous la forme d'un relief du porte-stérilet.

6. Le porte-stérilet selon l'une quelconque des revendications 4 ou 5, dans lequel la réglette (11) est située dans la partie du porte-stérilet qui reçoit le stérilet (1) et de préférence, dans la partie qui reçoit la partie de la gaine (3) qui comporte la butée réglable (4).

7. Le porte-stérilet selon l'une quelconque des revendications 1 à 6, dans lequel le porte-stérilet est biseauté dans la région du porte-stérilet qui reçoit l'extrémité (13) de la gaine.

8. Le porte-stérilet selon l'une quelconque des revendications 1 à 7, dans lequel le porte-stérilet a un relief à l'intérieur de la partie du porte-stérilet qui reçoit l'extrémité (13) de la gaine.

9. Le porte-stérilet selon l'une quelconque des revendications 1 à 8, dans lequel le porte-stérilet est en un matériau résilient, et de préférence transparent.

10. Le porte-stérilet selon l'une quelconque des revendications 4 à 9, dans lequel :
(a) le dispositif de fixation (8) est positionné vers le milieu du porte-stérilet ;
(b) la réglette (11) peut être lue de chaque côté de la gaine (3) lorsque la gaine (3) se trouve sur le porte-stérilet ; et
(c) la largeur du porte-stérilet est d'environ 1,5 à 8 cm.

11. Une combinaison comprenant un stérilet (1) et un porte-stérilet, caractérisée en ce que le stérilet (1) est un stérilet connu dans l'art et le porte-stérilet est un porte-stérilet selon l'une quelconque des revendications 1 à 10.

12. Une combinaison selon la revendication 11, dans laquelle le stérilet (1) est un stérilet comportant deux branches en porte-à-faux résilientes (2) recourbées en direction de la tige du stérilet.

13. La combinaison selon la revendication 11 ou 12, dans laquelle le stérilet (1) est fixé dans une gaine (3).

14. La combinaison selon l'une quelconque des revendications 11 à 13, dans laquelle le porte-stérilet est recouvert, de préférence d'une feuille détachable.
